# EUROPEAN PATENT APPLICATION

(11) **EP 4 606 899 A1**
(43) Date of publication of application: **27.08.2025**
(21) Application number: 23880117.9
(22) Date of filing: 12.10.2023
(51) Int. Cl.: C12N 15/74, C12N 15/70

(54) **EPISOMAL VECTOR OPERATING IN BACTEROIDES SPP**

(30) Priority: 17.10.2022 KR 20220133538; 11.10.2023 KR 20230135421
(71) Applicant: Korea Research Institute of Bioscience and Biotechnology, Daejeon 34141 (KR)
(72) Inventor: LEE, Dae Hee, Daejeon 34141 (KR); KIM, Tae Hyun, Daejeon 34141 (KR); KIM, Seong Keun, Daejeon 34141 (KR); WOO, Seung Gyun, Daejeon 34141 (KR); LEE, Seung Goo, Daejeon 34141 (KR)
(74) Representative: Ipsilon Lyon
(86) International application number: PCT/KR2023/015771
(87) International publication number: WO 2024/085539

(57) **Abstract**

The present invention relates to a vector that operates in strains of the *Bacteroides* genus, which are beneficial gut microbes. The vector carries the nucleic acid sequence of SEQ ID NO: 1 necessary for the transformation of Escherichia coli and *Bacteroides* strains, and the nucleic acid sequence of SEQ ID NO: 2 including the origin of replication for Bacteroides strains. By recombining the nucleic acid sequence of SEQ ID NO: 2 with that of SEQ ID NO: 1, the vector can stably exist and autonomously replicate within *Bacteroides* strains to maintain a high number of copies. Thus, the vector can be advantageously used as a gut biosensor or for manufacturing tool for microbiome-based therapeutic agents in the future.

## Description

### TECHNICAL FIELD

### [CROSS-REFERENCE TO RELATED APPLICATIONS]

This application claims priority from Korean Patent Application No. 10-2022-0133538, filed on October 17, 2022, the disclosure of which is incorporated herein by reference in its entirety.

In addition, this application claims priority from Korean Patent Application No. 10-2023-0135421, filed on October 11, 2023, the disclosure of which is incorporated herein by reference in its entirety.

The present invention relates to an episomal vector operating in a microorganism belonging to the genus *Bacteroides.*

### BACKGROUND ART

Recently, the consumption of probiotics, which are microorganisms that are beneficial to the body, such as lactic acid bacteria, is increasing. Probiotics are a general term for living microorganisms that are beneficial to the human body, and as is well known, examples thereof include *Bifidobacterium* and *Lactobacillus.* Prebiotics are non-digestible substances that help improve the intestinal environment by acting as food for microorganisms that help the growth and activity of beneficial intestinal microorganisms, and representative examples thereof include carbohydrates such as oligosaccharides and dietary fiber. Postbiotics are substances produced by microorganisms that have a wide range of effects on the human body and play a beneficial role in the human body, and mainly include enzymes and peptides. These prebiotics, probiotics, and postbiotics are not intended to treat a specific disease, but rather to help the composition and function of the human microbiome. Although there are a large number of these probiotics, accurate studies on which microorganisms are beneficial and which are not beneficial to which people are still lacking.

However, recently, studies are being conducted to overcome these uncertainties and improve probiotics by engineering them to be more precise to move beyond their auxiliary role in health and to act more actively in the diagnosis and treatment of diseases. As a representative example, medical microorganisms that may detect intestinal inflammation are being developed.

Among them, bacteria of the genus *Bacteroides* are dominant beneficial bacteria that account for 20 to 30% of the human intestinal microorganisms, and bacteria of the genus *Bacteroides* are expected to be widely utilized when they are developed as customized medical microorganisms that may diagnose or treat intestinal diseases. However, there is a problem that plasmids for *E. coli* or plasmids that may be applied to a wide range of strains do not replicate or operate in strains of the genus *Bacteroides,* and in the genus *Bacteroides,* only an integrative vector that is integrated into the genome of the strain and expressed may be used.

However, since the integrative vector is not efficient in gene expression, there is a need for studies on a vector system that may replicate well in bacteria of the genus *Bacteroides* and express a target gene in order to develop medical bacteria of the genus *Bacteroides.*

### DISCLOSURE OF THE INVENTION

### TECHNICAL PROBLEM

An object of the present invention is to provide an episomal vector that operates in a strain of the genus *Bacteroides* and a microorganism transformed with the vector.

### TECHNICAL SOLUTION

One aspect of the present invention provides an episomal vector containing a nucleic acid sequence of SEQ ID NO: 1 and a nucleic acid sequence of SEQ ID NO: 2.

Another aspect of the present invention provides a microorganism transformed with the episomal vector.

### ADVANTAGEOUS EFFECTS

An integrated vector that integrates a target gene into the chromosome of the strain and expresses the target gene was used as the conventional vector used in the strain of the genus *Bacteroides,* and an episomal vector that may be used in the strain of the genus *Bacteroides* is not known. In the case of the integrative vector, the target gene is integrated into the chromosome of the strain of the *Bacteroides* and expressed, and thus, insertional mutagenesis may occur or the copy number of the target gene may only be 1, resulting in inefficient expression of the target gene.

The vector of the present invention may replicate autonomously while existing in the cytoplasm without being integrated into the chromosome of the strain even after being introduced into the strain of the genus *Bacteroides,* which is the most dominant beneficial bacterium in the intestinal flora. Therefore, the vector of the present invention may be maintained at a high copy number even in the strain of the genus *Bacteroides,* which may improve the expression of the target gene.

In addition, the vector of the present invention has high stability even after being introduced into the strain, such that the number of plasmids does not decrease over time or may be maintained at a similar level.

However, the effects of the present application are not limited to the effects mentioned above, and other effects that are not mentioned may be obviously understood by those skilled in the art from the following description.

### BRIEF DESCRIPTION OF THE DRAWINGS

FIG. 1 illustrates a structure of an episomal vector of the present invention.
FIG. 2 illustrates the results of qPCR using an episomal vector of the present invention and an integrative vector as a control. FIG. 2A is a graph showing fluorescence signals according to a cycle targeting β-lactamase or mannanase, which are present in both the episomal vector and the integrative vector, FIG. 2B illustrates a melt curve that may be used to confirm whether non-specific amplification occurred by gradually increasing a temperature after performing qPCR for each target, and FIG. 2C illustrates the copy number of the episomal vector of the present invention.
FIG. 3 illustrates the results of comparing luminescence intensities of luciferase expressed for six types of constitutive promoters using the integrative vector or the episomal vector of the present invention.
FIG. 4 illustrates the results of measuring the expression level of Amuc_1102 by SDS-PAGE and western blot after transformation with the vector of the present invention or integrative vector, into which Amuc_1102 (28 kDa) protein gene is inserted.
FIG. 5 illustrates the results of measuring the expression level of Amuc_1100 by western blot after transformation with the vector of the present invention or integrative vector, into which Amuc_1100 (34 kDa) protein gene is inserted.
FIG. 6 illustrates the results of culturing in BHIS plate containing erythromycin to confirm whether the episomal vector of the present invention is effectively introduced and operates for various strains of the genus *Bacteroides.*
FIG. 7 illustrates the results of PCR amplification of the episomal vector to confirm whether the episomal vector is effectively introduced into various strains of the genus *Bacteroides.*
FIG. 8 illustrates the results of Western blot obtained by observing the expression of the Amuc_1102 secretory protein after expressing the Amuc_1102 protein using the episomal vector of the present invention in *B. fragilis, B. uniformis, B. ovatus,* and *B. vulgatus* strains.
FIG. 9 illustrates the results showing that, unlike the episomal vector of the present invention, since a strain transformed with a truncated episomal vector in which the sequence of SEQ ID NO. 2 is partially deleted does not survive in a selection plate, the nucleic acid sequence of SEQ ID NO. 2 is an essential configuration for functioning as a replication origin in a strain of the genus *Bacteroides.*
FIG. 10 illustrates a structure of an episomal vector containing thyA gene.

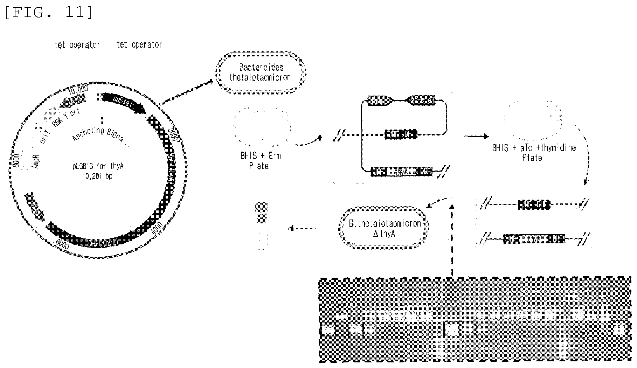

FIG. 11 illustrates a method for producing an auxotroph for thymidine.
FIG. 12 illustrates the results of qPCR in a thymidine auxotroph when the episomal vector containing the thyA gene is transformed into the strain.
FIG. 13 illustrates the results of comparing the stability of vectors in the strain when using an episomal vector containing the thyA gene and an episomal vector not containing the thyA gene as a control.

### BEST MODE FOR CARRYING OUT THE INVENTION

First, the terms used in the present specification are defined.

The term "vector" as used in the present specification refers to a DNA molecule used as an artificial carrier of a specific gene. The vector may express a target protein or target RNA in a host cell, a specific gene may be inserted into the vector through cell transformation, and the resulting vector may be delivered to a host cell so that it may be replicated in large numbers or may be used as a gene carrier that enables expression as a protein. The vector may be a genetic construct containing essential regulatory elements operably linked to enable expression of a genetic insert. An expression vector contains an expression control sequence, and as necessary, a signal sequence or leader sequence for membrane targeting or secretion, and may be produced in various ways depending on the purpose. The expression control sequence is a DNA sequence that controls expression of an operably linked polynucleotide sequence in a specific host cell. The expression control sequence contains a promoter for initiating transcription, an optional operator sequence for regulating transcription, a sequence encoding a suitable mRNA ribosome binding site, and a sequence for regulating the termination of transcription and translation. In addition, the expression vector may contain a selection marker for selecting a host cell containing the vector, and a replicable expression vector may contain a replication origin. The vector may contain a base sequence encoding various proteins known in the art, and the base sequence encoding the target protein is operably linked to a promoter.

The term "operably linked" as used in the present specification means that one nucleic acid fragment is linked to another nucleic acid fragment so that the function or expression thereof is affected by the other nucleic acid fragment. Alternatively, the term "operably linked" refers to a linkage that enables transcription or translation to produce a functional transcription or translation product. An operable linkage between a target gene and a vector may be produced using a genetic recombination technique well known in the art, and enzymes generally known in the art may be used in site-specific DNA cleavage and linkage.

The term "episomal vector" as used in the present specification is a non-viral and non-integrative vector that is distinct from an integrative vector, and refers to a vector that expresses a gene contained in the vector without being inserted into a chromosome. Since the episomal vector is not inserted into a chromosome of a host cell, the episomal vector is unlikely to cause insertional mutagenesis and may replicate and proliferate independently of the genome of the host cell.

Hereinafter, the present application will be described in detail.

### 1. Episomal vector of the present invention

One aspect of the present invention provides an episomal vector.

The episomal vector contains a replication origin (OriV) of *E. coli.* The replication origin of *E. coli* may be a specific nucleic acid sequence at which replication is initiated in *E. coli,* which confers the ability to replicate in *E. coli.* For example, a DNA replication origin of *E. coli* may be R6K or the like, but is not limited thereto, and may be used without limitation as long as it confers the ability to replicate in *E. coli.* Since the episomal vector contains the replication origin of *E. coli,* the episomal vector is easily recombined in *E. coli* and may be efficiently amplified.

The episomal vector contains at least one selection marker for selecting a transformed host cell. The selection marker may be an antibiotic resistance gene, a gene encoding a luminescent or fluorescent protein, or an essential nutrient producing gene. The antibiotic may be tetracycline, chloramphenicol, kanamycin, erythromycin, or ampicillin.

Since the antibiotic resistance gene confers antibiotic resistance to a transformed host cell, the transformed host cell may be selected by confirming whether the host cell survives in an environment containing antibiotics, and examples thereof include a gene encoding β-lactamase, a gene encoding chloramphenicol acetyltransferase, a tetA gene of class A (including RP1, RP4, or Tn1721 derivative), B (Tn10 derivative), and C (pSC101 or pBR322 derivative) encoding a tetracycline efflux system, and a Neo gene. Since the luminescent protein gene confers an activity that enables the transformed host cell to emit light, the transformed host cell may be selected by confirming whether the host cell emits light, and for example, the luminescent protein gene may be a luciferase gene, and since the fluorescent protein gene confers an activity that enables the transformed host cell to exhibit fluorescence, the transformed host cell may be selected by confirming whether the host cell exhibits fluorescence, and for example, the fluorescent protein gene may be a jellyfish green fluorescent protein (GFP) or red fluorescent protein (RFP) gene. The essential nutrient producing gene may be a gene involved in producing an amino acid, nucleoside, nucleotide, nucleic acid base, or protein.

The selection marker gene may be used to select a host cell into which the episomal vector is successfully introduced, and may be used according to the characteristics of the host cell into which the vector is introduced when used as a shuttle vector. For example, the selection marker gene may be a β-lactamase gene for selecting *E. coli,* or an erythromycin resistance gene for selecting a strain of the genus *Bacteroides.*

The episomal vector may contain a transfer origin (OriT). The transfer origin is a noncoding nucleic acid sequence, and is a region required for conjugation, which is a horizontal gene transfer method between microorganisms. During the conjugation process, DNA is transferred from a donor cell to a recipient cell through close contact between two cells. The transfer origin is a region where bacterial conjugation begins, and conjugation begins when a multi-protein complex called relaxosome is assembled at the transfer origin in Gram-negative bacteria. The transfer origin may be RP4, RK2, and the like, which are used for conjugation between *E. coli* and the genus *Bacteroides,* but is not limited thereto, and any transfer origin may be used without limitation as long as it enables gene transfer through conjugation between microorganisms. The episomal vector may be transformed through a conjugation process with *E. coli* as well as a general transformation method because it contains the transfer origin.

The episomal vector may contain genes of the replication origin, the selection marker, and the transfer origin of *E. coli,* and the replication origin, the selection marker, and the transfer origin of *E. coli* may be operably linked. Specifically, the episomal vector may contain a part of a pMM553 vector containing the replication origin of *E*. *coli* (R6K oriV), the selection marker gene of *E. coli* (β-lactamase gene), the selection marker gene of a strain of the genus *Bacteroides* (erythromycin resistance gene), and OriT (RP4), and more specifically, the nucleic acid sequence of SEQ ID NO: 1. In this case, the episomal vector may be prevented from being inserted into the genome of the host cell by removing NBU2 and an integration site involved in integration from the pMM553 vector.

In addition, the episomal vector may further contain a replication origin of the genus *Bacteroides,* and the replication origin of the genus *Bacteroides,* which is a specific nucleic acid sequence that initiates replication in a bacterium of the genus *Bacteroides,* may confer the ability to replicate in the bacterium of the genus *Bacteroides.* For example, the DNA replication origin of the bacterium of the genus *Bacteroides* may contain the nucleic acid sequence of SEQ ID NO: 2, but is not limited thereto, and any DNA replication origin may be used without limitation as long as it confers the ability to replicate in the bacterium of the genus *Bacteroides.* Since the nucleic acid sequence of the replication origin of the genus *Bacteroides* confers the ability to replicate in a strain of the genus *Bacteroides,* when the episomal vector containing the replication origin of the genus *Bacteroides* is introduced, the episomal vector may replicate autonomously in the *Bacteroides* cell into which the episomal vector is introduced. In particular, the nucleic acid sequence of SEQ ID NO: 2 may function as a replication origin by being contained in the episomal vector together with the nucleic acid sequence of SEQ ID NO: 1.

The nucleic acid sequence of SEQ ID NO: 2 may be inserted by replacing a gene involved in insertion or integration into the genome of the host cell in the pMM553 vector. Specifically, the 3' end of the nucleic acid sequence of SEQ ID NO: 1 and the 5' end of SEQ ID NO: 2, and the 5' end of the nucleic acid sequence of SEQ ID NO: 1 and the 3' end of SEQ ID NO: 2 may be linked, respectively, by cleaving the integrative vector pMM553 with restriction enzymes NotI and AgeI and then linking to the nucleic acid sequence of SEQ ID NO: 2 having both ends to which the cleavage sequences of the restriction enzymes are attached, respectively.

Since the episomal vector contains the nucleic acid sequence of SEQ ID NO: 2, a high copy number may be maintained even in a strain of the genus *Bacteroides,* such that the episomal vector may be used as an expression vector. In a case where a part of the nucleic acid sequence of SEQ ID NO: 2 is deleted and inserted in the episomal vector, the episomal vector may not replicate autonomously.

Accordingly, the episomal vector of the present invention may contain the nucleic acid sequence of SEQ ID NO: 1 and the nucleic acid sequence of SEQ ID NO: 2.

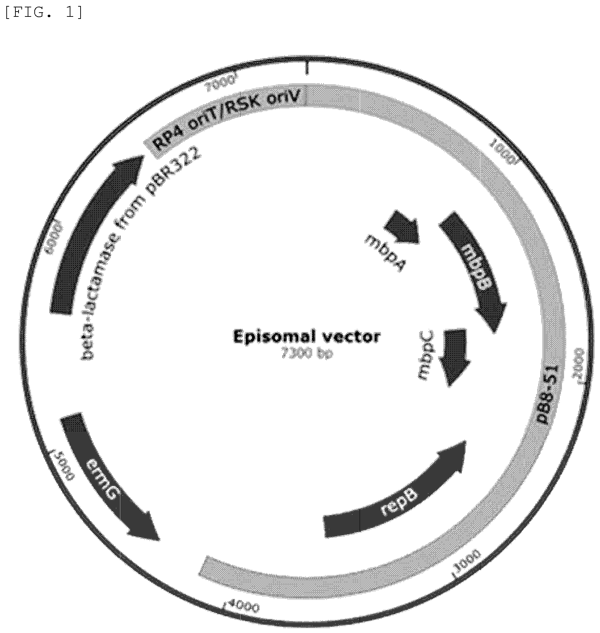

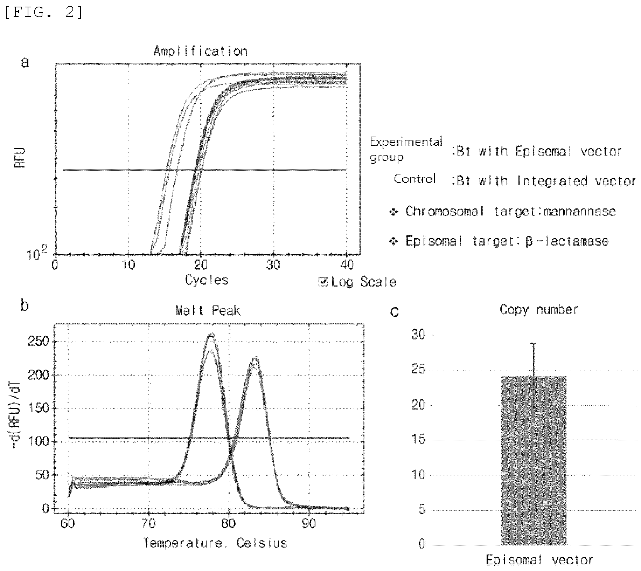

In a specific embodiment of the present invention, an episomal vector of SEQ ID NO: 3 containing a nucleic acid sequence of SEQ ID NO: 1 and a nucleic acid sequence of SEQ ID NO: 2 was produced (FIG. 1), and after introducing the episomal vector into a *Bacteroides thetaiotaomicron* strain and performing qPCR, it was confirmed that while a conventional integrative vector had a copy number of about 1, the episomal vector of the present invention had a copy number of about 24, confirming that the episomal vector is an episomal vector that replicates autonomously (FIG. 2).

In addition, the episomal vector of the present invention may further contain a gene involved in synthesis of essential nutrients.

The gene involved in the synthesis of the essential nutrients may be involved in a process of expressing proteins that synthesize the essential nutrients. The essential nutrients refer to nutrients that cannot be synthesized in the body in order to maintain growth or normal physiological functions, and may include essential amino acids, essential fatty acids, vitamins, nucleic acid DNA, and the like. Examples of the gene involved in the synthesis of the essential nutrients include a thymidine synthesis gene, a glutamate synthesis gene, a histidine synthesis gene, an arginine synthesis gene, and a threonine synthesis gene, and specifically, thyA, RacE, and YrpC, but are not limited thereto.

The gene involved in the synthesis of the essential nutrients may be operably linked to the episomal vector, and specifically, may be operably linked to the nucleic acid sequence of SEQ ID NO: 1 and the nucleic acid sequence of SEQ ID NO: 2, for example, may be located within the nucleic acid sequence of SEQ ID NO: 1, within the nucleic acid sequence of SEQ ID NO: 2, or between the nucleic acid sequence of SEQ ID NO: 1 and the nucleic acid sequence of SEQ ID NO: 2. However, the location of the gene involved in the synthesis of the essential nutrients is not limited thereto, and the gene involved in the synthesis of the essential nutrients may be included in a location that does not affect the function of the gene encoded by the nucleic acid sequence of SEQ ID NO: 1 or SEQ ID NO: 2.

In addition, the episomal vector may further contain a multiple cloning site (MCS).

The multiple cloning site is a site where the cleavage sites of several restriction enzymes are gathered, and refers to an arrangement of sequences recognized by restriction enzymes to make it easy to cleave or insert a gene fragment. The multiple cloning site may be a combination of at least one or more sequences recognized by restriction enzymes such as BglII, XhoI, ScaI, HindIII, EcoRI, PstI, SalI/AccI, KpnI, SacII, ApaI, S/XmaI, BamHI, ApaLI, PmII, BstZ17I, NruI, AscI, PvuI, FseI, AcII, BsiWI, BspEI, ClaI, ScaI, BsrGI, and SpeI. The multiple cloning site may be operably linked to the nucleic acid sequence of SEQ ID NO: 1 and the nucleic acid sequence of SEQ ID NO: 2, and may be located, for example, within the nucleic acid sequence of SEQ ID NO: 1, within the nucleic acid sequence of SEQ ID NO: 2, or between the nucleic acid sequence of SEQ ID NO: 1 and the nucleic acid sequence of SEQ ID NO: 2. However, the location of the multiple cloning site is not limited thereto, and the multiple cloning site may be included in a location that does not affect the function of the gene encoded by the nucleic acid sequence of SEQ ID NO: 1 or SEQ ID NO: 2.

In addition, the episomal vector may further contain a promoter that may operate in a host cell. The promoter may be operably linked to the nucleic acid sequence of SEQ ID NO: 1, the nucleic acid sequence of SEQ ID NO: 2, and the multiple cloning site, for example, the promoter may be operably linked to expression of a gene that is inserted between the nucleic acid sequence of SEQ ID NO: 1 and the multiple cloning site. Specifically, the promoter may be a promoter that may operate in a strain of the genus *Bacteroides.* For example, the promoter may be P_{BT1311}, P_{BfP1E6}, P_{BT3743}, P_{BT3744}, P_{cfiA}, P₁, P₂, P_{cepA}, P_{cfxA}, P_{chuR}, P_{susB}, P_{cusBC}, P_{BfP2E2}, P_{BfP2E3}, P_{BfP1E4}, P_{BfP5E4}, P_{BfP2E5}, P_{BfP4E5}, P_{AM1}, P_{AM2}, P_{AM3}, P_{AM4}, PBF2884, P_{BT3763}, P_{BT3334}, P_{BT0267}, _{PLacO13}, P_{LacO23}, P_{DX}, P_{BT1830}, P_{BT4615}, P_{rRNA}, P_{BA}, P1T_{DP}, or the like, but is not limited thereto, and may be used without limitation as long as it is a promoter that may operate in a strain of the genus *Bacteroides.*

In addition, the vector may further contain an initiation codon, a stop codon, a polyadenylation signal, a Kozak, an enhancer, a signal sequence for membrane targeting or secretion, an internal ribosome entry site (IRES), a transcription terminator, or any operator sequence for regulating transcription, which is a noncoding nucleic acid sequence that helps to promote or influence transcription, translation, or expression of a target gene.

### 2. Use of episomal vector of the present invention

Another aspect of the present invention provides a microorganism transformed with the episomal vector.

Still another aspect of the present invention provides a method for transforming a microorganism using the episomal vector.

The episomal vector of the present invention relates to a special type of plasmid, which has the characteristic of being able to replicate autonomously without being inserted into the chromosome even when transformed into *Bacteroides,* which is a host cell. Therefore, unlike the integrative vector conventionally used in a strain of the genus *Bacteroides,* there is no possibility of mutation due to insertion, and since the episomal vector replicates and proliferates independently of the chromosome, there is an effect of increasing the expression level of the target gene.

Therefore, the episomal vector of the present invention may be used for transforming a host microorganism with a target gene.

The target gene may be a gene encoding a protein that may confer or improve functions such as disease prevention, disease diagnosis, growth promotion, and immune enhancement, such as an antigen, an antibody, a receptor, a hormone, a cytokine, a regulatory protein, or a structural protein.

The transformation includes any method for introducing a foreign gene into a host cell, and may be performed by selecting a standard technique suitable for the host cell as known in the art. A transformation method includes, but is not limited to, electroporation, protoplast fusion, calcium phosphate precipitation, calcium chloride precipitation, stirring using silicon carbide fibers, PEG, dextran sulfate, lipofectamine, and the like, and any commonly known transformation method may be used.

In particular, as described above, in the case where the episomal vector of the present invention contains the gene involved in the synthesis of the essential nutrients, the vector of the present invention may be introduced into an auxotroph, which is a mutant strain that is unable to produce essential nutrients required for growth for transformation, and an essential nutrient synthesis protein is expressed in the strain, such that the essential nutrients may be synthesized, thereby enabling the growth of the auxotroph. The stability of the vector may be improved by using the gene involved in the synthesis of the essential nutrients and the auxotroph.

In a specific embodiment of the present invention, an episomal vector containing a nucleic acid sequence of SEQ ID NO: 11 was produced by inserting a thymidine synthesis gene containing a nucleic acid sequence of SEQ ID NO: 10 between a selection marker gene of *E. coli* (β-lactamase gene) and a selection marker gene of a strain of the genus *Bacteroides* (erythromycin resistance gene). A thymidine auxotroph was transformed with the episomal vector, and the number of plasmids in the transformed strain and the proportion of plasmid-containing strains over time were measured. As a result, it was confirmed that the number of plasmids was about 11, and a high proportion of the plasmid-containing strains was maintained even after transformation.

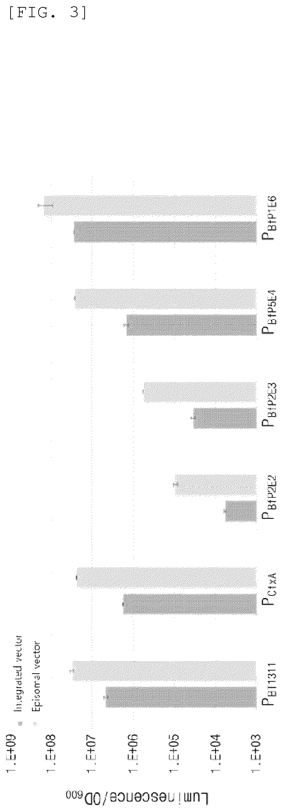

In a specific embodiment of the present invention, as a result of analyzing the expression of luciferase, which is a target gene, using the episomal vector of the present invention, it was confirmed that in a case where the episomal vector of the present invention was used, the amount of light emitted increased compared to a case where the integrative vector was used. In addition, as a result of analyzing expression of a gene encoding a secretory protein, which is a target gene, using the episomal vector of the present invention, it was confirmed that, in the case where the episomal vector of the present invention was used, the expression level increased compared to the case where the integrative vector was used. Through this, it was confirmed that in the case where the episomal vector of the present invention was used, the expression level of the target gene in the strain of the genus *Bacteroides* increased more effectively than in the case where the conventional integrative vector was used (see FIGS. 3 to 5).

In particular, the episomal vector may be used for transformation of a strain of the genus *Bacteroides.* The strain of the genus *Bacteroides* is an obligate anaerobic Gram-negative strain, which acts as a beneficial intestinal bacterium that metabolizes polysaccharides and oligosaccharides in mammalian organisms and provides nutrients and vitamins to a host and other intestinal microorganisms. In particular, the strain of the genus *Bacteroides* is a dominant beneficial bacterium that accounts for 20 to 30% of the human intestinal microorganisms, and 30 species or more strains of the genus *Bacteroides* have been discovered to date. For example, the strain of the genus *Bacteroides* may include, but is not limited to, strains of the genus *Bacteroides,* including *B. thetaiotaomicron, B. fragilis, B. uniformis, B. ovatus, B. vulgatus, B. xylanivsolvens, B. acidifaciens, B. plebeius, B. eggerthii,* and *B. dorei.*

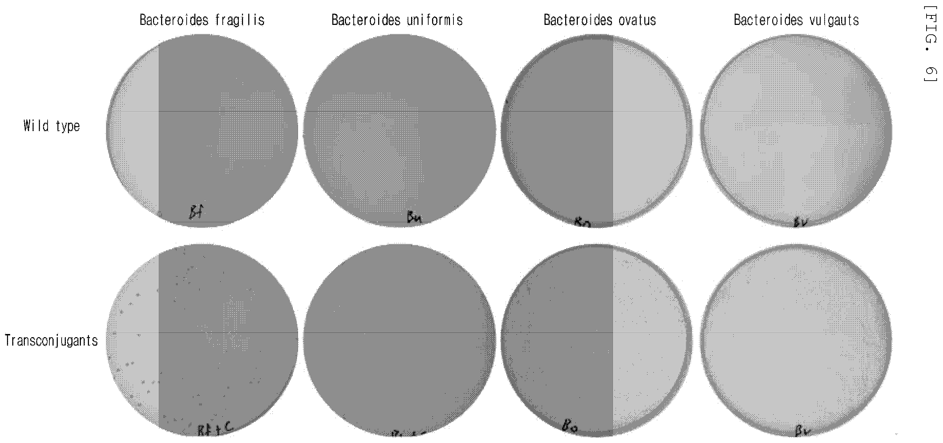

In a specific embodiment of the present invention, the episomal vector of the present invention was effectively introduced into strains of the genus *Bacteroides,* including *B. thetaiotaomicron, B. fragilis, B. uniformis, B. ovatus,* and *B. vulgatus,* and it was confirmed that it is an episomal vector that commonly operates in the strains of the genus *Bacteroides* (see FIGS. 6 to 8).

The transformed microorganism may be a strain of *E*. *coli* or the genus *Bacteroides.*

In one embodiment of the present invention, the transformed microorganism may be a strain having auxotrophy for essential nutrients.

In one embodiment of the present invention, the transformed microorganism may be a strain having auxotrophy for thymidine, and specifically, may be a strain of the genus *Bacteroides* having auxotrophy for thymidine.

Hereinafter, the present application will be described in detail with reference to examples.

However, the following examples are intended to illustrate the present application specifically, and the present application is not limited by the following examples.

### [Example 1]

### Design and production of episomal vector

An episomal vector capable of replicating autonomously in the genus *Bacteroides* was designed and produced.

Specifically, the recombinant vector of the present invention contains a nucleic acid sequence of SEQ ID NO: 1 and a nucleic acid sequence of SEQ ID NO: 2. The nucleic acid sequence of SEQ ID NO: 1 is a sequence containing a replication origin of *E. coli* (R6K oriV), a selection marker gene of *E*. *coli* (β-lactamase gene), a selection marker gene of a strain of the genus *Bacteroides* (erythromycin resistance gene), and OriT (RP4) required for conjugation of *E. coli* and a strain of the genus *Bacteroides* present in a pMM553 vector, and the nucleic acid sequence of SEQ ID NO: 2 is a nucleic acid sequence containing a replication origin of the genus *Bacteroides.* In addition, the nucleic acid sequence of SEQ ID NO: 2 contains the replication origin of *Bacteroides,* but has the characteristic that it cannot replicate autonomously and cannot be transmitted to other cells.

Accordingly, as illustrated in FIG. 1, the present inventors produced an episomal vector containing a nucleic acid sequence of SEQ ID NO: 3 by replacing the genes (P_{BT1311}, NanoLuc, Terminator, Integrase, and integration site) involved in integration into the genome of the host cell or in luminescence in the conventional pMM553 vector with the nucleic acid sequence of SEQ ID NO: 2, thereby reconstructing the vector so that it may be efficiently introduced into a strain of the genus *Bacteroides* and replicate autonomously within the strain.

Specifically, the pMM553 vector was cleaved with NotI and AgeI to remove unnecessary parts, such as the NBU2 integrase and attBT2 site, to obtain the nucleic acid sequence of SEQ ID NO: 1. The nucleic acid sequence of SEQ ID NO: 2 (4,154 bp) was synthesized in Macrogen Inc., PCR was performed using a primer capable of attaching the cleavage sequences of NotI and AgeI and the complementary wing sequences to both ends, and then, the nucleic acid sequence of SEQ ID NO: 2 was linked to the nucleic acid sequence of SEQ ID NO: 1, thereby producing an episomal vector containing a nucleic acid sequence of SEQ ID NO: 3.

### [Example 2]

### Confirmation of copy number of plasmid

In order to confirm whether the vector of the present invention produced in Example 1 is actually capable of replicating autonomously in a strain of the genus *Bacteroides,* the copy number of plasmid was confirmed in the strain of the genus *Bacteroides* transformed with the vector of the present invention.

Specifically, the vector of the present invention or the integrative vector pMM553 containing the nucleic acid sequence of SEQ ID NO: 1 as a control was first transformed into *E. coli* WM3064 strain, which is a donor strain, and cultured in LB plate, and a wild-type *Bacteroides thetaiotaomicron* strain (*B. thetaiotaomicron*)*,* which is a recipient strain, was cultured in BHIS plate, respectively.

The donor strain and the recipient strain were cultured so that an optical density of each bacterial solution was 0.2 to 0.3. 0.2 ml of the culture medium of the donor strain and 1.4 ml of the culture medium of the recipient strain were placed in tubes, respectively, and centrifuged at 7,200 g, the supernatant was removed, 0.2 ml of BHIS plate was added to each tube, and centrifugation was performed again under the same conditions. The supernatant of each strain was removed, 20 µl of BHIS was added, and the pellets of the donor strain and recipient strain were mixed. A sterilized filter with a pore size of 0.22 µm was attached to the BHIS plate containing 5% sheep blood, and the mixed solution of strains was inoculated. Aerobic culture was performed at 37°C for 20 hours to ensure that the mixed solution of strains was completely absorbed into the filter, and then, the filter was separated and suspended in 5 ml of PBS. 0.2 ml of the suspension was spread on BHIS plate containing erythromycin and cultured anaerobically for 3 days. As described above, the *Bacteroides thetaiotaomicron* strain was transformed by conjugation with *E*. *coli.*

A *Bacteroides thetaiotaomicron* transformant of each vector was cultured and diluted to an optical density of 0.5/ml, and centrifuged at 13,000 rpm for 1 minute, the supernatant was removed, centrifugation was performed again at 13,000 rpm for 1 minute using 1 ml of PBS, and then, the supernatant was removed again, thereby obtaining pellets. 1 ml of triple distilled water was added to the pellets, mixed, and heated for 10 minutes to obtain a genetic sample of the transformant.

5 ul (100 pmol) of a primer set targeting β-lactamase (Amp^{R}) gene contained in both the vector of the present invention and the pMM553 vector as a control and the mannanase gene (control) of the *Bacteroides* strain gRNA and KOD SYBR qPCR Mix (14 µl of distilled water, 25 µl of qPCR mix) were added to 1 µl of the total gene sample obtained as described above, and then, qPCR was performed by repeating 40 cycles of heating at 98°C for 10 seconds, 60°C for 10 seconds, and 68°C for 30 seconds. The sequences of the primers are shown in Table 1.

Thereafter, the temperature of the amplified target gene sample was gradually increased from 65°C to 95°C, and the fluorescence signal was measured to confirm the melt curve to confirm whether non-specific amplification occurred by each primer set.

**[Table 1]**

| Primer | SEQ ID NO: | Sequence (5'→3') |
|---|---|---|
| Bt_mannanase forward | 4 | CATCGTTCGTCAGCAGTAACA |
| Bt_mannanase reverse | 5 | CCAAGAAAAAGGGACAGTGG |
| pTH310_qPCR forward | 6 | ggataaagttgcaggaccac |
| pTH310_qPCR reverse | 7 | tcagcaataaaccagccagc |

As a result, as illustrated in FIG. 2A, it was confirmed that amplification occurred well for each target. Specific Cq values are shown in Table 2. In the case of the pMM553 vector as a control, the Cq values of the β-lactamase gene were 19.28, 19.11, and 19.35, and in the case of the vector of the present invention, the Cq values were 15.58 and 15.22.

**[Table 2]**

| Target gene | Vector | Cq |
|---|---|---|
| α-mannanase | Example 1 | 15.58 |
| | | 15.22 |
| | pMM553 (Control) | 19.28 |
| | | 19.11 |
| | | 19.35 |
| β-lactamase | Example 1 | 20.02 |
| | | 20.04 |
| | pMM553 (Control) | 19.23 |
| | | 19.11 |
| | | 19.26 |

In addition, as illustrated in FIG. 2B, the verification result melt curve also showed that only two peaks for the two targets (β-lactamase gene and mannanase gene) appeared, confirming that non-specific amplification did not occur. As a result of calculating the copy number of plasmid by raising the absolute value obtained by subtracting the Cq value of gRNA mannanase from the Cq value of each vector to 2, as illustrated in FIG. 2C, the vector of the present invention had a copy number of about 24, while the copy numbers of the pMM553 vector were 1.04, 1, and 1.06. Through these experimental results, it was confirmed that the vector of the present invention is an episomal vector that may be successfully introduced into a strain of the genus *Bacteroides,* may replicate autonomously within the strain, and is an efficient expression vector having a copy number of 20 or more.

### [Example 3]

### Confirmation of effect of increasing gene expression through vector of the present invention

### [3-1] Confirmation of increased gene expression using luciferase

It was confirmed whether the episomal vector of the present invention may actually express a target gene in a microorganism of the genus *Bacteroides* and whether the gene expression level is increased compared to the existing integrative vector.

The episomal vector produced in Example 1 was cleaved with restriction enzymes EcoRI and SacI. After synthesizing an insert sequence, PCR was performed using primers that may attach a sequence complementary to the cleavage sequence of EcoI to one end of the insert sequence and a sequence complementary to the cleavage sequence of SacI to the other end. The insert sequence was synthesized using six types of promoters (P_{BT1311}, P_{cfxA}, P_{BfP2E2}, P_{BfP2E3}, P_{BfP5E4}, and P_{BfP1E6}) constitutively expressed in *Bacteroides thetaiotaomicron* strains and NanoLuc (luciferase) as a reporter gene. After cloning the episomal vector and the insert sequence through Gibson assembly, a completed vector was finally confirmed through Sanger sequencing.

After transforming a *Bacteroides* strain with the vector thus produced or the integrative vector pMM553 as a control in the same manner as in Example 2, the luminescence of the expressed luciferase was analyzed.

As a result, as illustrated in FIG. 3, in the case of the episomal vector of the present invention, regardless of the type of promoter, the amount of light emitted increased by at least 5 times compared to the existing integrative vector pMM553, and in particular, in the case of the most excellent promoter P_{BfP5E4}, the amount of light emitted increased by 17 times or more compared to the pMM553 vector.

### [3-2] Confirmation of increased gene expression using secretory protein

Furthermore, the expression increase effect of the vector of the present invention was confirmed using a secretory protein Amuc_1100 (34 kDa) or Amuc_1102 (28 kDa).

Specifically, the genes of the two target proteins were inserted between the 3' end of SEQ ID NO: 1 and the 5' end of SEQ ID NO: 2 using two types of promoters (P_{BT1311} or P_{BfP1E6}) to produce a recombinant vector in the same manner as in Example [3-2]. In the case of the pMM553 as a control, the same promoter and target protein gene were inserted into the cloning site to produce a recombinant vector, and each of the recombinant vectors was transformed into the *Bacteroides thetaiotaomicron* strain. SDS-PAGE and western blot were performed on each of a cell lysate obtained by dissolving the pellets obtained by centrifuging the culture medium of the *Bacteroides* strain and a supernatant obtained by precipitating the protein with trichloroacetic acid (TCA) to measure an expression level of the protein.

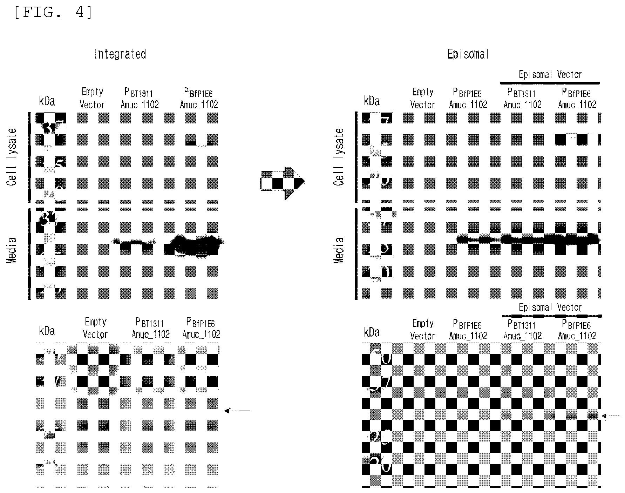

As a result, in the case of Amuc_1102 (28 kDa), as illustrated in FIG. 4, the expression level increased when P_{BfP1E6} was used as a promoter compared to when P_{BT1311} was used. In addition, it was confirmed that in the case where the vector of the present invention (Episomal vector) was used, the Amuc_1102 band appeared more distinctly than in the case where the pMM553 vector (Integrated) as a control was used.

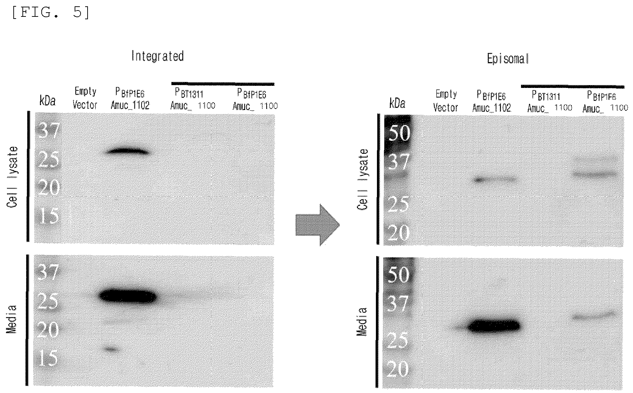

In addition, in the case of the Amuc_1100 (34 kDa) protein, as illustrated in FIG. 5, when the protein was expressed using the pMM553 vector, no band was observed in the Western blot, whereas when the vector of the present invention was used, a band for Amuc_1100 was clearly observed.

Through the experimental results as described above, it was found that the vector of the present invention may increase the expression of the target gene in a *Bacteroides* strain more effectively than the integrative vector pMM553 conventionally used for transformation of a strain of the genus *Bacteroides.*

### [Example 4]

### Confirmation of applicability to various strains of the genus Bacteroides

In order to confirm whether the episomal vector of the present invention commonly operates well in other strains belonging to the genus *Bacteroides,* in addition to *B. thetaiotaomicron* used in Examples 2 and 3, for four types of strains of the genus *Bacteroides,* it was confirmed whether each transformed strain of the genus *Bacteroides* was capable of replicating autonomously within the strain and forming colonies on selection plates.

Specifically, four types of strains of the genus *Bacteroides,* such as *B. fragilis, B. uniformis, B. ovatus,* and *B. vulgatus,* were transformed using the same method as described above. In order to confirm whether each strain of the genus *Bacteroides* was successfully transformed, it was confirmed that, when the wild-type and transformed strains of each strain were spread and cultured on BHIS plate containing erythromycin, only the strain transformed with the episomal vector of the present invention formed colonies, as illustrated in FIG. 6.

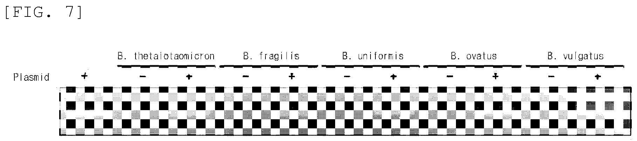

In addition, PCR was performed using primers specific for the episomal vector on the genomes obtained by centrifugation of five types of transformed strains of the genus *Bacteroides,* including the *Bacteroides thetaiotaomicron* strain. PCR was also performed on each wild-type strain in the same manner. As a result, as illustrated in FIG. 7, a band for the episomal vector was observed only in the strain transformed with the vector of the present invention, confirming that the strain was successfully transformed and the vector was replicated.

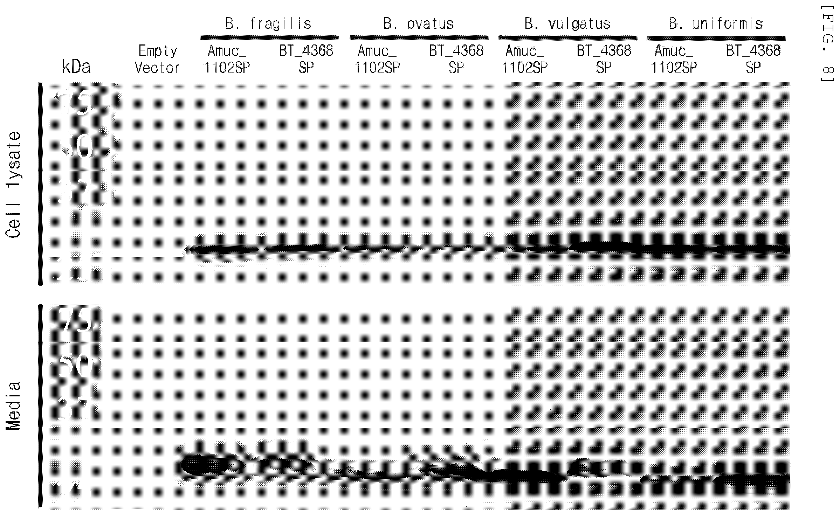

In addition, the target gene Amuc_1102 was expressed in the four types of strains of the genus *Bacteroides* using the episomal vector of the present invention in the same manner as in Example 3-2, and then, the secretion and expression of the Amuc_1102 protein was observed through Western blot. As a result, as illustrated in FIG. 8, it was confirmed that Amuc_1102 was secreted and expressed in all the four types of strains.

Through these experimental results, it was found that the episomal vector of the present invention may successfully transform various strains of the genus *Bacteroides* and maintain a high copy number by replicating autonomously within the strain, and thus, is an episomal vector that may commonly operate in the strains of the genus *Bacteroides.*

The present invention relates to a vector capable of operating efficiently in a strain of the genus *Bacteroides,* which is a beneficial bacterium accounting for about 20% or more of the intestinal flora, and may effectively increase the expression of a foreign gene because a high copy number may be maintained in the strain of the genus *Bacteroides.* Therefore, the episomal vector of the present invention may be usefully used to produce an intestinal biosensor or a microbiome therapeutic agent.

### [Example 5]

In order to confirm whether the nucleic acid sequence of SEQ ID NO: 2 of the episomal vector of the present invention is an essential component for the episomal vector to operate in a strain of the genus *Bacteroides, Bacteroides* was transformed using a sequence in which a part of the nucleic acid sequence of SEQ ID NO: 2 was deleted.

Specifically, only the repB sequence, which functions as a replication origin in a strain of the genus *Bacteroides* in the nucleic acid sequence of SEQ ID NO: 2, was synthesized to produce a truncated episomal vector using the same method as in Example 1. A strain of the genus *Bacteroides* was transformed using the episomal vector of the present invention or the truncated episomal vector in the same manner as in Example 2.

As a result, as illustrated in FIG. 8, the strain transformed with the truncated episomal vector formed colonies on the mating plate, but did not form colonies on the BHIS selection plate containing erythromycin, and it was confirmed that only the strain of the genus *Bacteroides* transformed with the episomal vector of the present invention formed colonies on the selection plate.

It was found through this that the nucleic acid sequence of SEQ ID NO: 2 is an essential component for operating as the replication origin of the vector in the strain of the genus *Bacteroides.*

### [Example 6]

### Effect of improving stability of episomal vector using auxotroph

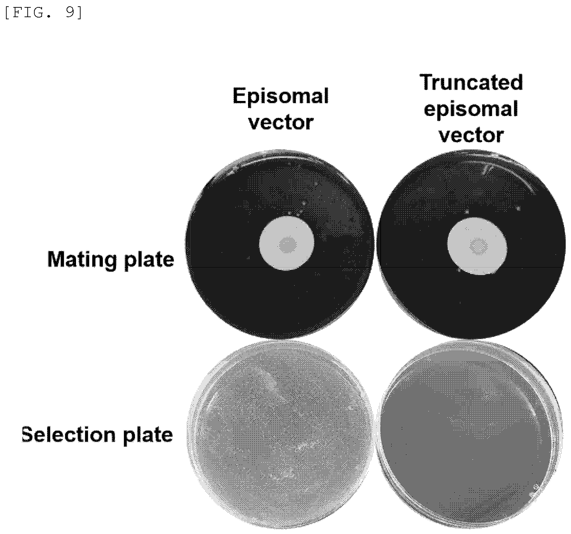

In order to confirm whether the stability of the episomal vector of the present invention may be improved when using an auxotroph and a gene involved in synthesis of essential nutrients, the following experiments were performed. First, in the episomal vector of Example 1, the thyA (thymidylate synthase, BT_2047) gene of SEQ ID NO: 10 was additionally inserted between the selection marker gene of *E*. *coli* (β-lactamase gene) and the selection marker gene of the strain of the genus *Bacteroides* (erythromycin resistance gene), thereby producing an episomal vector containing a nucleic acid sequence of SEQ ID NO: 11 (hereinafter, referred to as pTH310t2 vector) (FIG. 9). Specifically, the pTH310t2 vector was obtained and completed by PCR of the backbone of the pTH310 plasmid and PCR of the thyA gene in a wild-type *Bacteroides thetaiotaomicron* strain through Gibson assembly.

Hereinafter, the stability of the episomal vector of Example 1 and the stability of the pTH310t2 vector were compared. Specifically, a thymidine auxotroph was produced and transformed with the pTH310t2 vector, and the copy number of plasmid was measured, which was compared with the copy number of plasmid in the strain transformed with the vector of Example 1.

### [6-1] Production of thymidine auxotroph

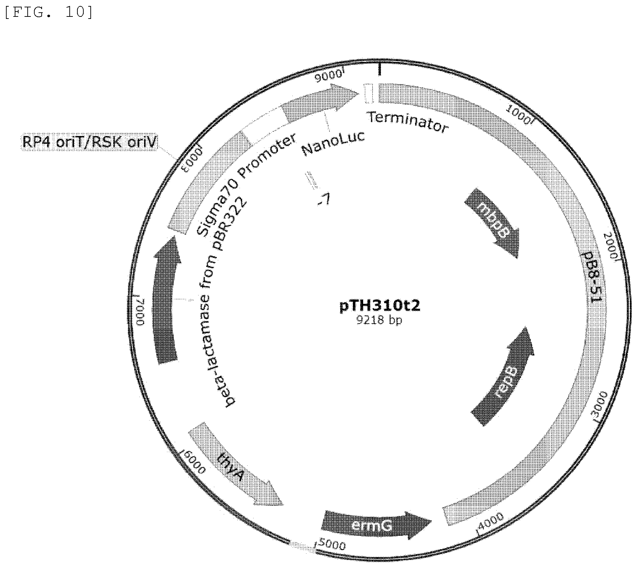

The thymidine auxotroph refers to a strain in which the thyA gene is knocked out (KO) and thus cannot synthesize thymidine within the body, and thus has difficulty surviving unless the insufficient substance is provided from outside. The plasmid for the knockout was constructed using pLGB13 (source: mBio (2019) 10(4), 01762-19) as the backbone. Specifically, 2.5 KB sequences before and after thyA were cloned into pLGB13 as UHF and DHF (upstream or downstream homology flank), respectively. Thereafter, the vector cloned as described above was introduced into the wild-type *Bacteroides thetaiotaomicron* strain, and the strain colonies were obtained by culturing on a plate containing Erm antibiotics. From the obtained colonies, colonies in which the plasmid was inserted into the chromosome were selected, and the selected colonies were obtained and spread on a plate containing anhydrotetracycline (aTc) and thymidine instead of antibiotics for 3 minutes. By confirming the size of the protein band through PCR for the colonies generated on the plate, a strain that produced an appropriate PCR band was obtained and used as a thymidine auxotroph of the present invention (FIG. 10).

### [6-2] Confirmation of copy number of plasmid

The copy number of plasmid was confirmed in the strain of the genus *Bacteroides* transformed using each of pMM553, the vector of Example 1, and the pTH310t2 vector by the following method. Specifically, a strain of the genus *Bacteroides* was transformed using pMM553 and the vector of Example 1, and when the pTH310t2 vector was used, the auxotroph of Example 6-1 was transformed. In addition, in order to prepare a DNA sample for measuring the copy number of plasmid, a DNA sample was obtained through washing twice by PCR using QIAamp DNA Mini Kit, and the DNA sample obtained as above was used in 1 ul qPCR to measure the copy number of plasmid.

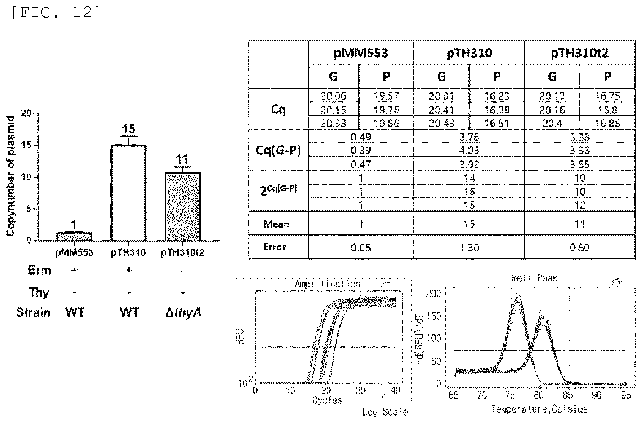

As a result, as illustrated in FIG. 12, in the case where the pTH310t2 vector was used, the copy number of plasmid was measured to be about 11, and the qPCR results and melt curve results also showed similar levels to those in the case where the vector of Example 1 was used, confirming that there were no particular abnormalities. Through these experimental results, it was found that the pTH310t2 vector of the present invention is an episomal vector that may be successfully introduced into a strain of the genus *Bacteroides,* may replicate autonomously within the strain, and is an efficient expression vector having a copy number of 11 or more.

### [6-3] Confirmation of effect of improving stability of pTH310t2 vector

In order to compare the stability of the vector of Example 1 and the stability of the pTH310t2 vector, a proportion of plasmid-bearing cells appearing as the microbial strain transformed with the vector was subcultured was measured. First, the wild-type *Bacteroides thetaiotaomicron* strain was transformed with the vector of Example 1 and subcultured every 24 hours in BHIS plate without antibiotics (1 Passage = 24 h). After the subculture, the remaining cells were diluted and spread on an erythromycin antibiotic-containing plate and an erythromycin antibiotic-free plate, respectively, and cultured anaerobically for 3 days. The number of colonies containing the plasmid was measured in the two types of plates, and a ratio of the number of colonies in the antibiotic-containing plate to the number of colonies in the antibiotic-free plate was calculated. Next, the auxotroph of Example 6-1 was transformed with the pTH310t2 and subcultured every 24 hours in BHIS plate without antibiotics (1 Passage = 24 h). After the subculture, the remaining cells were diluted and spread on a thymidine-containing plate and a thymidine-free plate, respectively, and then cultured anaerobically for 3 days. Thereafter, the number of colonies containing the plasmid was measured in the two types of plates, and a ratio of the number of colonies in the thymidine-free plate to the number of colonies in the thymidine-containing plate was calculated.

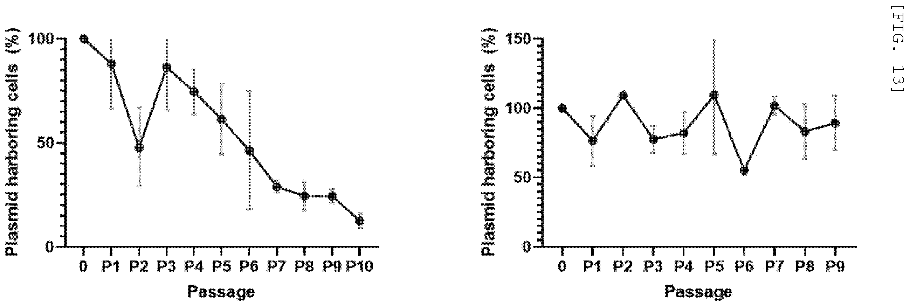

As a result, as illustrated in FIG. 13, in the case where the vector of Example 1 was used, a proportion of cells containing the plasmid decreased over time, and in the case where the pTH310t2 and the auxotroph were used, it was confirmed that a proportion of cells close to 100% maintained the plasmid even over time. Therefore, it was found that the stability of the vector was improved by using the pTH310t2 vector and the auxotroph.

Although the representative embodiments of the present invention have been described above as examples, the scope of the present invention is not limited to the specific embodiments described above, and those skilled in the art will appreciate that modifications are possible within the scope as described in the claims of the present application.

## Claims

1. An episomal vector comprising a nucleic acid sequence of SEQ ID NO: 1 and a nucleic acid sequence of SEQ ID NO: 2.

2. The episomal vector of claim 1, wherein the episomal vector is used for transformation of a strain of the genus *Bacteroides.*

3. The episomal vector of claim 1, further comprising a multiple cloning site located between the nucleic acid sequence of SEQ ID NO: 1 and the nucleic acid sequence of SEQ ID NO: 2.

4. The episomal vector of claim 2, further comprising a promoter inserted between the nucleic acid sequence of SEQ ID NO: 1 and a cloning site.

5. The episomal vector of claim 4, wherein the promoter is a promoter that is operable in the genus *Bacteroides.*

6. The episomal vector of claim 4, wherein the promoter is selected from the group consisting of P_{BT1311}, P_{BfP1E6}, P_{BT3743}, P_{BT3744}, P_{cfiA}, P₁, P₂, P_{cepA}, P_{cfxA}, P_{chuR}, P_{susB}, P_{cusBC}, P_{BfP2E2}, P_{BfP2E3}, P_{BfP1E4}, P_{BfP5E4}, P_{BfP2E5}, P_{BfP4E5}, P_{AM1}, P_{AM2}, P_{AM3}, P_{AM4}, P_{BF2884}, P_{BT3763}, P_{BT3334}, P_{BT0267}, P_{LacO13}, P_{LacO23}, P_{DX}, P_{BT1830}, P_{BT4615}, P_{rRNA}, P_{BA}, and P1T_{DP}.

7. The episomal vector of claim 1, further comprising a gene involved in synthesis of essential nutrients.

8. The episomal vector of claim 7, comprising a nucleic acid sequence of SEQ ID NO: 11.

9. A microorganism transformed with the episomal vector of any one of claims 1 to 8.

10. The microorganism of claim 9, wherein the microorganism is a strain of the genus *Bacteroides.*

11. The microorganism of claim 9, wherein the strain of the genus *Bacteroides* has auxotrophy for essential nutrients, and the episomal vector contains a gene involved in synthesis of the essential nutrients.

12. A method for transforming a microorganism using the episomal vector of any one of claims 1 to 8.
